# EUROPEAN PATENT SPECIFICATION

(11) **EP 1 954 227 B1**
(45) Date of publication and mention of the grant of the patent: **22.07.2009**
(21) Application number: 06837004.8
(22) Date of filing: 06.11.2006
(51) Int. Cl.: A61F 9/007

(54) **INTRAOCULAR SHUNT DEVICE**
INTRAOKULARER SHUNT
DISPOSITIF DE DERIVATION INTRAOCULAIRE

(30) Priority: 08.11.2005 US 269371
(43) Date of publication of application: 13.08.2008
(73) Proprietor: BAUSCH & LOMB INCORPORATED, Rochester, NY 14604-2701 (US)
(72) Inventor: LEVY, Brian, Rochester, New York 14618 (US)
(74) Representative: Vossius & Partner
(86) International application number: PCT/US2006/043251
(87) International publication number: WO 2007/056325

(56) References cited:
- US-A1- 2002 026 200
- US-A1- 2005 049 578
- US-A1- 2005 090 807

## Description

### Field of Invention

The present invention relates to intraocular treatment devices, and more particularly to controllable shunts for controlling intraocular pressure.

### Background of the Invention

Glaucoma is a pathological condition of the eye often resulting in elevated intraocular pressure. If the intraocular pressure remains elevated for a long enough period of time, total vision loss occurs. The blindness that results from glaucoma involves both central and peripheral vision and has a major impact on an individual's ability to lead an independent life. Because glaucoma is a major cause of blindness, glaucoma is a significant public health problem.

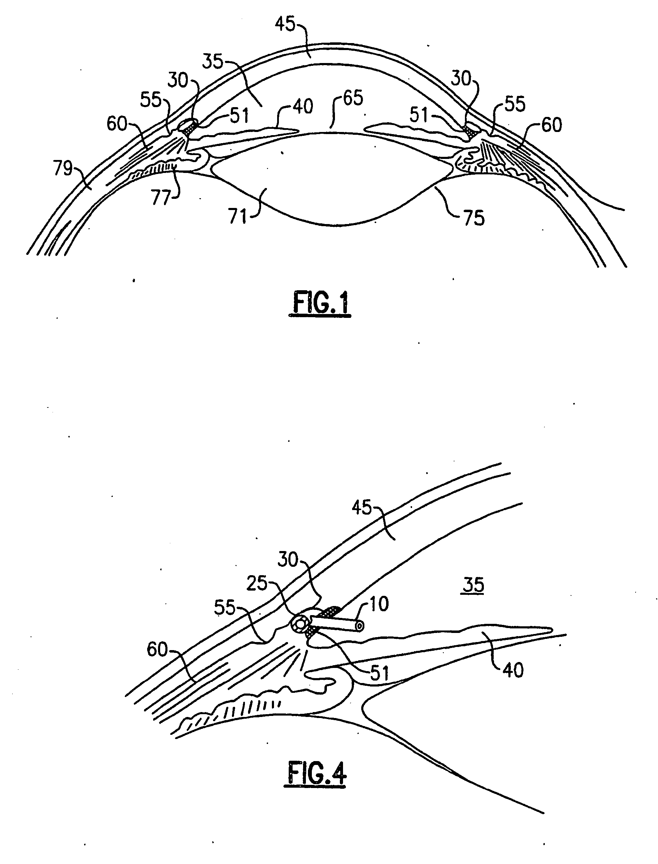

The following explanation of glaucoma is given with reference to the schematic illustration of the eye in FIG. 1. High pressure develops in an eye as a result of glaucoma because of an intraocular fluid imbalance. The pertinent fluid, known as aqueous humor, is formed in the posterior chamber 75 of the eye by the ciliary body at a fairly constant rate of about 2.5 microliters per minute. The aqueous fluid passes from the posterior chamber around the lens, through the pupillary opening 65 in the iris 40 and into the anterior chamber 35 of the eye. Once in the anterior chamber, the fluid drains out of the eye through two different routes. In a first route (referred to as the "uveoscleral route") the fluid percolates between muscle fibers 77 of the ciliary body. This route accounts for approximately ten percent of the aqueous outflow in humans. The primary route for aqueous outflow (referred to as the "canlicular route") involves the trabecular meshwork 51 and Schlemm's canal 30.

The trabecular meshwork and Schlemm's canal are located at the junction between the iris 40 and the sclera 79. This junction is commonly referred to as "the angle." The trabecular meshwork is a wedge-shaped structure that runs around the circumference of the eye. It is composed of collagen beams arranged in a three-dimensional sieve-like structure. The beams are lined with a monolayer of cells called trabecular cells. The spaces between the collagen beams are filled with an extracellular substance that is produced by the trabecular cells. These cells also produce.enzymes that degrade extracellular material. Schlemm's canal is adjacent to the trabecular meshwork. The outer wall of the trabecular meshwork coincides with the inner wall of Schlemm's canal. Schlemm's canal is a tube-like structure that runs around the circumference of the cornea.

The aqueous fluid travels through the spaces between the trabecular beams, across the inner wall of Schlemm's canal into the canal, through a series of about 25 collecting channels that drain from Schlemm's canal and into the episcleral venous system 60. In a normal situation, aqueous fluid production is equal to aqueous fluid outflow and intraocular pressure remains fairly constant in the 15 to 21 mmHg range. In glaucoma, the resistance through the canalicular outflow system is abnormally high, thus causing internal fluid imbalance.

In primary open angle glaucoma, which is the most common form of glaucoma, the abnormal resistance is believed to be along the outer aspect of trabecular meshwork and the inner wall of Schlemm's canal. It is believed that an abnormal metabolism of the trabecular cells leads to an excessive build up of extracellular materials or a build up of abnormally "stiff' materials in this area. Primary open angle glaucoma accounts for approximately eighty-five percent of all glaucoma. Other forms of glaucoma (such as angle closure glaucoma and secondary glaucomas) also involve decreased outflow through the canalicular pathway but the increased resistance is from other causes such as mechanical blockage, inflammatory debris, cellular blockage, etc.

With the increased resistance, the aqueous fluid builds up in the anterior chamber because it cannot exit fast enough to maintain the intraocular fluid balance. As mentioned above, as the fluid builds up, the intraocular pressure (IOP) increases. The increased IOP compresses the axons in the optic nerve (which carries visual information from the eye to the brain) and also may compromise the vascular supply to the optic nerve. While research is investigating ways to protect the nerve from an elevated pressure, the only therapeutic approach currently available for glaucoma is to reduce the intraocular pressure.

Conventional therapy involves a stepwise procedure. Typically, topical or oral medication is the first step of treatment. The medication has many serious side effects including: heart failure, respiratory distress, hypertension and depression. When the medication fails to adequately control the pressure, laser trabeculoplasty is often performed. If laser trabeculoplasty does not reduce the pressure enough, a trabeculectomy is performed, in which a hole is made in the sclera and angle region to allow aqueous fluid to leave the eye. The trabeclectomy is associated with many problems, including scarring and infection that results from bacteria entering the eye through the hole in the sclera.

When trabeculectomy doesn't successfully lower the eye pressure, the next surgical step may be insertion of an aqueous shunt device. In some conventional shunt devices, a silicone tube is attached at one end to a plastic (polypropylene or other synthetic) plate, which is sewn into the surface of the eye. The tube is inserted into the eye through a hole. The external portion of the tube is covered with either donor sclera or pericardium, and the conjunctiva is replaced and the incision is closed.

With such aqueous diversion devices, aqueous drains out of the eye through the silicone tube to the surface of the eye. Most of the problems that have developed with such shunt devices and procedures have occurred because aqueous fluid is drained from inside of the eye to the surface of the eye.

In many glaucoma patients, the resistance problem that causes increased intraocular pressure lies between Schlemm's canal and the anterior chamber. The canal itself, the collecting channels and the episcleral venous system all are functional. As a result, some conventional shunts have been configured to function by directing aqueous flow into Schlemm's canal rather than outside of the eye. Some such devices have included a pump, e.g. US-2002-0026200, which is considered as closest prior art. However, such devices have not provided suitable flow control.

### Summary

Aspects of the present invention are directed to a shunt device providing improved flow control. In one embodiment the shunt comprises a conduit sized to fit within Schlemm's canal, the conduit having a proximal end, a distal end, and an inlet intermediate the proximal end and the distal end; and a Venturi feature configured and arranged within the conduit so as to be capable of controlling flow from the inlet into the conduit. The shunt may further comprise a pressure sensor capable of measuring pressure within an eye, coupled to the Venturi feature, the Venturi feature being configured to control flow from the inlet into the conduit in response to a pressure measurement from the sensor. The Venturi feature may be electronically or magnetically actuatable in response to the pressure measurement. The sensor may be adapted to measure pressure continuously or intermittently.

The shunt may include an activation device configured to activate the Venturi feature and thereby control flow from the inlet to the conduit. The activation device may be connected to at least one of the conduit and the inlet. The activation device may be adapted to electrically or magnetically actuate the Venturi feature. The activation device may be adapted to control flow from the inlet into the conduit in response to a pressure measurement from a sensor. The Venturi feature may comprise one leaf, two leaves or more. Alternatively, the Venturi feature may be rotationally continuous (e.g., funnel-shaped).

The inlet may extend from the conduit or may simply be an opening in the conduit. In embodiments where the inlet extends from the conduit, the inlet is selected to have a length sufficient to extend into the anterior chamber of an eye when the shunt is implanted in Schlemm's canal.

The shunt may comprise a pump configured and arranged to control the flow in the conduit. In such embodiments, the shunt may comprise a pressure sensor capable of measuring pressure in the eye, the pressure sensor being coupled to the pump, and the pump being adapted to control flow from the inlet into the conduit in response to a pressure measurement from the sensor. The pump may be connected to the conduit. The pump may be separate from the conduit. The pump may be a peristaltic pump.

Another aspect not forming part of the invention is directed to a method of controlling a pressure in an anterior chamber of an eye, comprising providing in a Schlemm's canal of the eye a conduit having a Venturi feature disposed therein, and controlling flow along the Venturi feature, thereby achieving a pressure proximate an inlet from the anterior chamber into the Schlemm's canal. The method may further comprise a step of actuating the Venturi feature. The method may further comprise pumping fluid along the Venturi feature.

The term "Venturi feature" as used herein includes any suitable structure extending from the conduit and capable of producing a pressure change in response to fluid flow along the feature and thereby altering flow through the inlet. A Venturi feature may be actuatable or non-actuatable as described hereinbelow.

### Brief Description of the Drawings

Illustrative, non-limiting embodiments of the present invention will be described by way of example with reference to the accompanying drawings, in which the same reference number is used to designate the same or similar components in different figures, and in which:
FIG. 1 is a schematic illustration of certain anatomic features of the human eye;
FIG. 2A is a perspective view of an embodiment of a shunt according to aspects of the present invention;
FIG. 2B is a cross sectional side view of the embodiment of the present invention illustrating further aspects of the shunt shown in FIG. 1A;
FIG. 3 is a cross sectional side view of another embodiment of the present invention; and
FIG. 4 is a schematic illustration showing the surgical placement of an exemplary embodiment of the present invention.

### Detailed Description

Aspects of the present invention are directed to a shunt, comprising: a conduit sized to fit within Schlemm's canal, the conduit having a proximal end, a distal end, and an inlet intermediate the proximal end and the distal end; and a Venturi feature configured and arranged within the conduit so as to be capable of controlling flow from the inlet into the conduit.

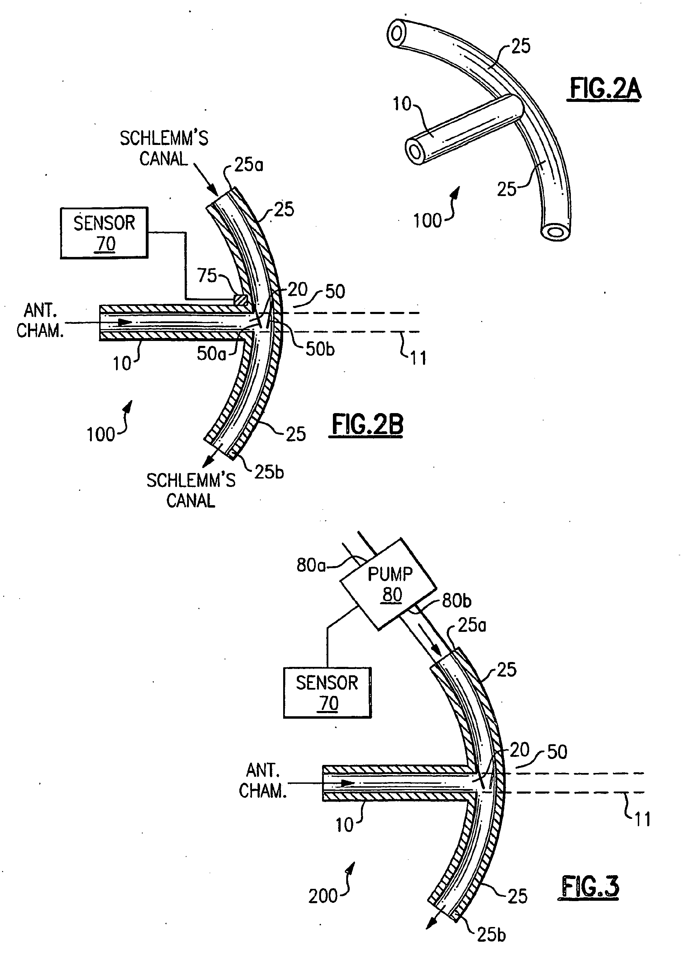

FIG. 2A is a schematic, perspective view of an embodiment of a shunt 100 according to aspects of the present invention including a Venturi feature (not shown). Shunt 100 is comprised of two portions, a conduit 25 and an inlet 10. FIG. 2B is a cross sectional view of shunt device 100 illustrating the internal fluid communication path. FIG. 2B also illustrates Venturi feature 50 disposed in the shunt to control fluid flow from the anterior chamber of an eye into Schlemm's canal. During operation of the device, conduit 25 is disposed in a portion of Schlemm's canal, and inlet 10 is exposed to the anterior chamber to divert fluid from the anterior chamber into Schlemm's canal as determined by Venturi feature 50.

In the illustrated embodiment, Venturi feature 50 may be configured and arranged in conduit 25 in any suitable manner such that actuation of the Venturi feature affects the amount of flow from the anterior chamber into inlet 10 and then into conduit 25. In the illustrated embodiment, Venturi feature 50 is disposed at least partially within the flow path 11 from inlet 10 as indicated by dashed lines.

It is to be appreciated that, according to the Venturi effect, flow through conduit 25 from proximal end 25a toward distal end 25b results in a flow on the interior side of Venturi feature 50 (i.e., the radially inward side of Venturi feature 50). Such flow results in a relatively low pressure region being formed in proximity to Venturi feature 50, thereby affecting flow into conduit 25 from the anterior chamber via inlet 10. As Venturi feature 50 is actuated to achieve more constricted flow through conduit 25, the pressure in proximity to Venturi feature 50 is reduced and flow from the anterior chamber via inlet 10 is increased. Correspondingly, as Venturi feature 50 is activated to achieve less constricted flow through conduit 25, the pressure in proximity to Venturi feature 50 is increased and flow into conduit 25 from the anterior chamber via inlet 10 is decreased.

For example, the Venturi feature may comprise a first leaf 50a and a second leaf 50b, where first leaf 50a is at least partially within flow path 11. In some embodiments, Venturi feature 50 may comprise only a single leaf 50a (i.e., leaf 50b may be omitted). In other embodiments, Venturi feature 50 may comprise a rotationally continuous structure, such as a funnel-shaped structure (e.g., a cone) having a channel formed therethrough where flow through conduit 25 may pass. In embodiments including a funnel-shaped structure, the structure may be made of compressible material, or the funnel-shaped structure may comprise flexible regions such that the cone is capable of being activated to constrict flow through conduit 25 and thereby control flow form the anterior chamber in the manner described above. In the present embodiment, control of fluid from the anterior chamber may be achieved using any suitable Venturi feature configuration by actuating Venturi feature 50 to increase and decrease pressure in proximity to Venturi feature 50.

Venturi feature 50 may be actuatable by any suitable technique. For example, an activation device 75 may be disposed proximate Venturi feature 50. The activation device 75 may, for example, comprise a plate capable of maintaining an electric potential relative Venturi feature 50 so that activation device 75 may attract or repulse Venturi feature 50. Accordingly, Venturi feature 50 may be controlled to achieve greater or lesser flow through conduit 25, and thereby control flow from inlet 10 into conduit 25. It is to be appreciated that, by selecting a suitable material and geometry for the Venturi feature, a selected amount of resilience of the Venturi feature can be attained such that the Venturi feature reaches an equilibrium for a given amount of attractive or repulsive force in response to activation device 75, thereby controlling the flow along conduit 25.

Alternatively, activation device 75 may provide a variable magnetic field (e.g., the activation device may comprise an electromagnet) and Venturi feature 50 may be made of a material capable of responding to the magnetic field such that the Venturi feature may increase or decrease flow through conduit 25. It is to be appreciated that activation device 75 may be located inside, outside or within conduit 25; and device 75 may extend completely around conduit 25 or may only extend a limited portion of the channel. The activation device may be powered by a suitable power source, such as a battery source or an inductive energy source.

In some embodiments, shunt 100 is coupled to a sensor 70 capable of measuring pressure in the anterior chamber of the eye; and activation device 75 is adapted to control Venturi feature 50 in response to the measured pressure. For example, in response to an undesirably high anterior chamber pressure, the Venturi feature may be operated as described above to increase flow from the anterior chamber into Schlemm's canal; and in response to an undesirably low anterior chamber pressure, the Venturi feature may be operated as described above to decrease flow from the anterior chamber into Schlemm's canal.

Sensor 70 may measure anterior chamber pressure continuously or intermittently. Additionally, activation device 75 may be operated to activate the Venturi feature continuously in response to measured pressure or intermittently. Examples of suitable pressure sensors are given in U.S. Pat. No. 5,431,057, issued June 11, 1995 to Zimmer, et al., U.S. Pat. Nos. 5,005,577, issued April 9, 1991 to Frenkel and U.S. Patent No. 6,443,893, issued September 3, 2002 to Schnakenburg, et al. A processor (not shown) may be included to receive pressure information from sensor 70 and to provide a control signal to activation device 75 to control thereby the Venturi feature.

In embodiments where pressure is measured intermittently and/or activation device 75 is operated intermittently, actuation of Venturi feature 50 can provide a constant level of actuation between measurements and/or actuations. In some embodiments, sensor 70 is capable of providing pressure measurements to an instrument outside of (i.e., remote from) a patient's body such that anterior chamber pressure may be monitored. Additionally, in some embodiments, Venturi feature 50 is actuatable from outside of a patient's body independently of or based on pressure measurements. Any suitable telemetric devices may be used to achieve such remote measurement and/or control.

Conduit 25 may have any suitable length, and external or internal shape. Conduit 25 may be of sufficient length to extend from the junction with inlet 10 through any suitable portion of the entire circumference of Schlemm's canal. In some embodiments, conduit 25 is selected to have length of about 6 mm. For example, the internal and or external shape of conduit 25 may be round, oval, rectangular or triangular. Typically the external shape will be selected such that conduit 25 fills Schlemm's canal so that all fluid flowing through Schlemm's canal will flow through conduit 25. Further details of a suitable conduit structures are given in U.S. Patent No. 6,450,984, issued September 17, 2002, to Lynch.

In the illustrated embodiment, inlet 10 joins the conduit 25 at an angle sufficient to allow the placement of inlet 10 within the anterior chamber of the eye when conduit 25 is oriented in Schlemm's canal. Inlet 10 may be selected to have a sufficient length, about 0.1 to 3.0 mm or about 2.0 mm, to extend from its junction with conduit 25 into Schlemm's canal towards the adjacent space of the anterior chamber. While many geometries can be used for channeling aqueous humor, an internal diameter of between about 0.1 and 0.5 mm, preferably 0.20 mm for a shunt having a round conduit shape. Although inlet 10 was discussed above and illustrated in FIGs. 2A and 2B as having a length extending into the anterior chamber, it is to be appreciated that in some embodiments, inlet 10 may simply be an opening in conduit 25 exposed to anterior chamber to permit fluid to enter conduit 25.

Shunt 100 can be fabricated from a material that will be compatible with the tissues and fluids with which it is in contact. It is preferable that the shunt not be absorbed, corroded, or otherwise structurally compromised during its presence in situ. Moreover, it is also preferable that the eye tissues and the aqueous fluid remain non-detrimentally affected by the presence of the implanted device. A number of materials are available to meet the engineering and medical specifications for the shunts. In the exemplary embodiments of the present invention, shunt 100 is constructed of a biologically inert, flexible material such as silicone or similar polymers. Alternate materials might include, but are not limited to, thin-walled Teflon, polypropylene, other polymers or plastics, metals, or some combination of these materials. The material may contain a therapeutic agent deliverable to the adjacent tissues.

Because the nature of the iris 40 is such that it tends to comprise a plurality of rather flaccid fimbriae of tissue, it is desirable to avoid said fimbriae from being drawn into the lumen of a shunt, thus occluding the shunt. Therefore, inlet 10 may contain a plurality of fenestrations in a lateral surface of the inlet as well as the end of the end of the inlet to allow fluid ingress, arranged to prevent occlusion by the adjacent iris.

Furthermore, inlet 10 may be positioned sufficiently remotely from the iris 40 to prevent interference therewith. Shunt 100 may contain one or more unidirectional valves to prevent backflow into the anterior chamber from Schlemm's canal.

The conduit 25 may have a pre-formed curve to approximate the 6.0 mm radius of Schlemm's canal in a human eye. Such a pre-formed curvature may not be implemented when a flexible material is used to construct the shunt device 100.

FIG. 3 is a cross sectional view of another embodiment according to aspects of the present invention. Shunt 200 is similar to shunt 100 described above, other than a pump 80 is added which controls flow through conduit 25 in response to a measured anterior chamber pressure measured by sensor 70. Because many aspects of shunt 200 are the same as those described above with reference to FIGs. 2A and 2B, only the aspects unique to shunt 200 or aspects that were not discussed above will be described below. It is to be appreciated that in the embodiment of FIG. 3 Venturi feature 50 need not be actuatable (i.e., it may be non-actuatable). In such embodiments, a pressure change proximate inlet 10 is established by increasing or decreasing the flow rate through conduit 25.

Pump 80 is inserted along Schlemm's canal in a manner such that fluid from the portion of Schlemm's canal enters an input 80a of pump 80 and flows to an output 80b of pump 80 into conduit 25 which is disposed in Schlemm's canal. Pump 80 is a pump capable of controlling the flow rate of fluid through Schlemm's canal (i.e., flow rate may be increased or decreased relative to the natural Schlemm's canal flow rate). Pump 80 may be connected to conduit 25; alternatively, pump 80 may be separate from conduit 25, but fluidly coupled to conduit 25. Pump 80 may comprise any suitable pump for controlling flow along Schlemm's canal. For example, the pump may be a peristaltic pump (e.g., a pump as described in U.S. Patent No. 6,852,500 or U.S. Patent Applic. US2002/0013545, both to Soltanpour, et al; or U.S. Pat. No. 6,408,878 to Unger et al.). Alternatively, the pump may be a pump as described in U.S. Patent No. 6,699,211 to Savage.

As described above, the flow rate that is established in conduit 25 affects the pressure proximate to Venturi feature 50, and thereby affects the flow rate of fluid flowing into input 10 from the anterior chamber. Accordingly, by controlling flow rate using pump 80, it is possible to control flow rate from the anterior chamber, and to thereby control the pressure within the anterior chamber.

FIG. 4 illustrates the placement of the exemplary embodiment of a shunt in an eye according to aspects of the present invention. FIG. 4 illustrates the anterior chamber 35, Schlemm's canal 30, the iris 40, cornea 45, trabecular meshwork 51, collecting channels 55, pupil 65 and lens 71. It should be noted that the inventive device is designed so that placement of conduit 25 within Schlemm's canal 30 results in an orientation of the inlet 10 within the anterior chamber 35 between the iris 40 and the inner surface of the cornea 45.

Any suitable surgical procedure may be used to insert shunts as described herein. An exemplary surgical procedure to insert at least some shunt device according to aspects of the present invention includes an approach through a conjunctival flap. A partial thickness scleral flap is then created and dissected half-thickness into clear cornea. The posterior aspect of Schlemm's canal is identified and the canal is entered posteriorly. The anterior chamber may be deepened with injection of a viscoelastic and a miotic agent. The inlet of the shunt is then inserted through the inner wall of Schlemm's canal and trabecular meshwork into the anterior chamber within the angle between the iris and the cornea. In some cases, as incision may be needed from Schlemm's canal through the trabecular meshwork into the anterior chamber to facilitate passage of the proximal portion therethrough. One arm of the conduit (having proximal end 25a) is grasped and threaded into Schlemm's canal. In a similar fashion, the other arm of the conduit (having distal end 25b) may be inserted into Schlemm's canal in the opposing direction from the first arm. The scleral flap and conjunctival wound are closed in a conventional manner.

Having thus described the inventive concepts and a number of exemplary embodiments, it will be apparent to those skilled in the art that the invention may be implemented in various ways, and that modifications and improvements will readily occur to such persons. Thus, the embodiments are not intended to be limiting and presented by way of example only. The invention is limited only as required by the following claims.

## Claims

1. A shunt, comprising:
a conduit sized to fit within Schlemm's canal, the conduit having a proximal end, a distal end, and an inlet intermediate the proximal end and the distal end; and
a Venturi feature arranged within the conduit and controlling flow from the proximal end to the distal end the Venturi feature being disposed in the flow path of the inlet so as to be capable of controlling flow from the inlet into the conduit.

2. The shunt of claim 1, further comprising a pressure sensor capable of measuring pressure within an eye, coupled to the Venturi feature, the Venturi feature configured to control flow from the inlet into the conduit in response to a pressure measurement from the sensor.

3. The shunt of claim 2, wherein the Venturi feature is electronically actuatable in response to the pressure measurement.

4. The shunt of claim 2, wherein the Venturi feature is magnetically actuatable in response to the pressure measurement.

5. The shunt of claim 2, wherein the sensor is adapted to measure pressure continuously.

6. The shunt of claim 2, wherein the sensor is adapted to measure pressure intermittently.

7. The shunt of claim 1, further comprising an activation device configured to activate the Venturi feature and thereby control flow from the inlet to the conduit, and preferably comprising at least one of the following features:
wherein the activation device is connected to at least one of the conduit and the inlet,
wherein the activation device is adapted to electrically actuate the Venturi feature, and
wherein the activation device is adapted to magnetically actuate the Venturi feature.

8. The shunt of claim 7, further comprising a pressure sensor capable of measuring pressure within an eye, coupled to the activation device, whereby the activation device is adapted to control flow from the inlet into the conduit in response to a pressure measurement from the sensor.

9. The shunt of claim 1, wherein the Venturi feature comprises at least one leaf, and preferably, wherein the Venturi feature comprises at least two leaves.

10. The shunt of claim 1, wherein the inlet extends from the conduit.

11. The shunt of claim 10, wherein the inlet is selected to have a length sufficient to extend into the anterior chamber of an eye when the shunt is implanted in Schlemm's canal.

12. The shunt of claim 1, wherein the Venturi feature has a rotationally continuous shape, and preferably
wherein the Venturi feature is funnel-shaped.

13. The shunt of claim 1, further comprising a pump configured and arranged to control the flow in the conduit.

14. The shunt of claim 13, further comprising a pressure sensor capable of measuring pressure in the eye, the pressure sensor being coupled to the pump, and the pump being adapted to control flow from the inlet into the conduit in response to a pressure measurement from the sensor, and preferably comprising at least one of the following features:
wherein the pump is connected to the conduit,
wherein the pump is separate from the conduit, and
wherein the pump is a peristaltic pump.

## Patentansprüche

1. Shunt, der aufweist:
eine Röhre, die so bemessen ist, daß sie in den Schlemm-Kanal paßt, wobei die Röhre ein proximales Ende, ein distales Ende und einen Einlaß zwischen dem proximalen Ende und dem distalen Ende aufweist; und
ein Venturi-Merkmal, das in der Röhre angeordnet ist und den Fluß vom proximalen Ende zum distalen Ende steuert, wobei das Venturi-Merkmal im Durchflußweg des Einlasses angeordnet ist, um geeignet zu sein, den Fluß vom Einlaß in die Röhre zu steuern.

2. Shunt nach Anspruch 1, der ferner einen Drucksensor aufweist, der zur Druckmessung in einem Auge geeignet ist, der mit dem Venturi-Merkmal gekoppelt ist, wobei das Venturi-Merkmal konfiguriert ist, als Reaktion auf eine Druckmessung vom Sensor den Fluß vom Einlaß in die Röhre zu steuern.

3. Shunt nach Anspruch 2, wobei das Venturi-Merkmal als Reaktion auf die Druckmessung elektronisch ansteuerbar ist.

4. Shunt nach Anspruch 2, wobei das Venturi-Merkmal als Reaktion auf die Druckmessung magnetisch ansteuerbar ist.

5. Shunt nach Anspruch 2, wobei der Sensor eingerichtet ist, den Druck kontinuierlich zu messen.

6. Shunt nach Anspruch 2, wobei der Sensor eingerichtet ist, den Druck intermittierend zu messen.

7. Shunt nach Anspruch 1, der ferner eine Aktivierungsvorrichtung aufweist, die konfiguriert ist, das Venturi-Merkmal zu aktivieren und **dadurch** den Fluß vom Einlaß in die Röhre zu steuern, und der vorzugsweise mindestens eines der folgenden Merkmale aufweist:
wobei die Aktivierungsvorrichtung mit mindestens einem der Röhre und des Einlasses verbunden ist,
wobei die Aktivierungsvorrichtung eingerichtet ist, das Venturi-Merkmal elektrisch anzusteuern, und
wobei die Aktivierungsvorrichtung eingerichtet ist, das Venturi-Merkmal magnetisch anzusteuern.

8. Shunt nach Anspruch 7, der ferner einen Drucksensor aufweist, der zur Druckmessung in einem Auge geeignet ist, der mit der Aktivierungsvorrichtung gekoppelt ist, wodurch die Aktivierungsvorrichtung eingerichtet ist, als Reaktion auf eine Druckmessung vom Sensor den Fluß vom Einlaß in die Röhre zu steuern.

9. Shunt nach Anspruch 1, wobei das Venturi-Merkmal mindestens ein Blatt aufweist, und vorzugsweise wobei das Venturi-Merkmal mindestens zwei Blätter aufweist.

10. Shunt nach Anspruch 1, wobei sich der Einlaß von der Röhre erstreckt.

11. Shunt nach Anspruch 10, wobei der Einlaß so ausgewählt ist, daß er eine Länge aufweist, die ausreicht, sich in die anteriore Kammer eines Auges zu erstrecken, wenn der Shunt im Schlemm-Kanal implantiert ist.

12. Shunt nach Anspruch 1, wobei das Venturi-Merkmal eine rotationsstetige Form aufweist, und vorzugsweise wobei das Venturi-Merkmal trichterförmig ist.

13. Shunt nach Anspruch 1, der ferner eine Pumpe aufweist, die konfiguriert und angeordnet ist, den Fluß in der Röhre zu steuern.

14. Shunt nach Anspruch 13, der ferner einen Drucksensor aufweist, der zur Druckmessung im Auge geeignet ist, wobei der Drucksensor mit der Pumpe gekoppelt ist und die Pumpe eingerichtet ist, als Reaktion auf eine Druckmessung vom Sensor den Fluß vom Einlaß in die Röhre zu steuern, und der vorzugsweise mindestens eines der folgenden Merkmale aufweist:
wobei die Pumpe mit der Röhre verbunden ist,
wobei die Pumpe von der Röhre getrennt ist, und
wobei die Pumpe eine Peristaltikpumpe ist.

## Revendications

1. Dérivation, comprenant :
un conduit dimensionné pour s'ajuster dans le canal de Schlemm, le conduit ayant une extrémité proximale, une extrémité distale et un orifice d'entrée entre l'extrémité proximale et l'extrémité distale ; et
un système à Venturi aménagé dans le conduit et régulant l'écoulement de l'extrémité proximale vers l'extrémité distale, la particularité Venturi étant disposée dans le trajet d'écoulement de l'orifice d'entrée de façon à pouvoir réguler l'écoulement de l'orifice d'entrée dans le conduit.

2. Dérivation selon la revendication 1, comprenant en outre un capteur de pression capable de mesurer la pression dans un oeil, couplé au système à Venturi, le système à Venturi étant configuré pour réguler l'écoulement de l'orifice d'entrée dans le conduit en réponse à une mesure de pression du capteur.

3. Dérivation selon la revendication 2, dans laquelle le système à Venturi peut être électroniquement actionné en réponse à la mesure de pression.

4. Dérivation selon la revendication 2, dans laquelle le système à Venturi peut être magnétiquement actionné en réponse à la mesure de pression.

5. Dérivation selon la revendication 2, dans laquelle le capteur est adapté pour mesurer la pression en continu.

6. Dérivation selon la revendication 2, dans laquelle le capteur est adapté pour mesurer la pression par intermittence.

7. Dérivation selon la revendication 1, comprenant en outre un dispositif d'activation configuré pour activer le système à Venturi et réguler ainsi l'écoulement de l'orifice d'entrée au conduit, et comprenant de préférence au moins l'une des particularités suivantes :
le dispositif d'activation est relié à au moins l'un du conduit et de l'orifice d'entrée,
le dispositif d'activation est adapté pour actionner électriquement le système à Venturi, et
le dispositif d'activation est adapté pour actionner magnétiquement le système à Venturi.

8. Dérivation selon la revendication 7, comprenant en outre un capteur de pression capable de mesurer la pression dans un oeil, couplé au dispositif d'activation, le dispositif d'activation étant adapté pour réguler l'écoulement de l'orifice d'entrée dans le conduit en réponse à une mesure de pression du capteur.

9. Dérivation selon la revendication 1, dans laquelle le système à Venturi comprend au moins une lamelle, et de préférence, dans laquelle le système à Venturi comprend au moins deux lamelles.

10. Dérivation selon la revendication 1, dans laquelle l'orifice d'entrée s'étend à partir du conduit.

11. Dérivation selon la revendication 10, dans laquelle l'orifice d'entrée est sélectionné pour avoir une longueur suffisante de sorte à s'étendre dans la chambre antérieure d'un oeil lorsque la dérivation est implantée dans le canal de Schlemm.

12. Dérivation selon la revendication 1, dans laquelle le système à Venturi a une forme continue en rotation, et de préférence dans laquelle le système à Venturi a une forme d'entonnoir.

13. Dérivation selon la revendication 1, comprenant en outre une pompe configurée et agencée pour réguler l'écoulement dans le conduit.

14. Dérivation selon la revendication 13, comprenant en outre un capteur de pression capable de mesurer la pression dans l'oeil, le capteur de pression étant couplé à la pompe, et la pompe étant adaptée pour réguler l'écoulement de l'orifice d'entrée dans le conduit en réponse à une mesure de pression du capteur, et comprenant de préférence au moins l'une des particularités suivantes :
la pompe est reliée au conduit,
la pompe est séparée du conduit, et
la pompe est une pompe péristaltique.
